# EUROPEAN PATENT SPECIFICATION

(11) **EP 2 768 562 B1**
(45) Date of publication and mention of the grant of the patent: **03.01.2018**
(21) Application number: 12780697.4
(22) Date of filing: 17.10.2012
(51) Int. Cl.: A61B 5/00, A61B 5/01, A61B 5/087, A61M 16/08, A61M 16/00, A61M 16/10, A61M 16/04, A61M 16/16, A61B 5/097

(54) **SYSTEM FOR CONTROLLING DELIVERY OF RESPIRATORY GAS**
SYSTEM ZUR STEUERUNG DER VERABREICHUNG VON ATEMGAS
SYSTÈME DE RÉGLAGE DE L'ADMINISTRATION DE GAZ RESPIRATOIRE

(30) Priority: 21.10.2011 GB 201118204
(43) Date of publication of application: 27.08.2014
(73) Proprietor: Intersurgical AG, Vaduz (LI)
(72) Inventor: MILLER, Andrew, Wokingham, Berkshire RG41 2RZ (GB)
(74) Representative: Adamson Jones
(86) International application number: PCT/EP2012/070586
(87) International publication number: WO 2013/057146

(56) References cited:
- EP-A1- 1 206 922
- EP-A2- 1 306 098
- WO-A1-2011/008339
- DE-A1-102009 053 067
- US-A1- 2007 107 736
- US-A1- 2008 078 388
- US-A1- 2009 101 153
- US-A1- 2010 319 702
- US-A1- 2011 144 527

## Description

The present invention relates to respiratory circuits and, more particularly, to a system which allows for the control of one or more properties of the gas delivered via the respiratory circuit.

The artificial respiration of a patient requires the delivery of a gas under pressure to a patient's airway. The respiratory gas is regulated by a ventilator and passed to a patient interface via a set of tubes and associated devices, often referred to as a patient circuit.

During mechanical ventilation, a patient's nose is bypassed, and as such it is essential that the humidity of the air is maintained at a sufficiently high level. It is conventional in such systems to provide a humidification device, by which the moisture content of the gas to be inspired by a patient is increased. An evaporator may be used for this purpose, within which the inspiratory gas is passed over a heated water reservoir such that water vapour is carried by the gas to the patient. Such an arrangement also has the effect of warming the gas flow.

For the purpose of humidification, it is generally preferable to heat the water reservoir in the humidifier to an elevated temperature to promote evaporation and thus raise the moisture content of the respiratory gas stream. Accordingly a conventional heater for this purpose may operate to maintain the water reservoir at a predetermined elevated temperature in order to ensure that the relative humidity of the gas stream is maintained at least at a desired, constant level. Such a fixed operating condition is typically set using a manually operated dial.

Over-heating of the gas flow is however undesirable and can cause discomfort for the patent. In extreme circumstances a significantly over-heated and moisture-laden gas flow can scald the patient. Accordingly it has previously been proposed to monitor the temperature of the gas flow leaving the humidifier using a sensor and to control the rate of heating of the water reservoir based on a desired gas delivery temperature to the patient.

However the applicant has determined that the various different scenarios in which respiratory apparatus is used can lead to situations in which respiratory gas is not optimally conditioned using conventional equipment.

US2008/078,388 discloses a patient interface having an integrated system for communicating data to a ventilator.

US2010/319,702 discloses a method and system for determining tracheal and location information for a tracheal tube.

WO2011/008339 discloses a wireless gas flow-powered sensor system for a breathing assistance system.

It is an aim of the present invention to provide a system and associated apparatus which offer an improved degree of control over the conditioning of respiratory gas supplied to a patient.

According to one aspect of the invention there is provided a respiratory gas delivery system comprising a wearer interface through which respiratory gas is supplied to a wearer's airway, the wearer interface comprising a sensor for measuring the core body temperature of the wearer, a ventilator, and a humidification device for conditioning the respiratory gas upstream of the interface in a direction of gas flow to the wearer, the humidification device comprising a reservoir of water, and a heater for transfer of heat energy to the reservoir of water, characterised in that the humidification device comprises an inlet for receiving respiratory gases from the ventilator, and a controller, wherein the controller is arranged to receive data signals form the core body temperature sensor and to control operation of the heater based on said received data signals in order to achieve a desired temperature and/or humidification of the respiratory gas supplied to the wearer..

Additionally or alternatively, the wearer interface may comprise a further sensor. The further sensor may be arranged for measuring the temperature of the gas flow through a gas passage in the wearer interface. Additionally or alternatively, the wearer interface may comprise a gas flow or pressure sensor for the gas flow through the wearer interface.
In one embodiment, the body temperature sensor may be provided remotely of the wearer interface. Such sensor options may include an in-ear sensor or a skin temperature sensor. If provided remotely of the wearer interface, the sensor may be connected thereto, for example by one or more wires. Thus a temperature sensor can communicate with the controller via said wired connection.
The humidification device typically comprises a gas flow inlet and outlet to allow passage of gas over the reservoir, typically via an internal chamber which is part-filled with water.
The controller may control the degree or rate of humidification of respiratory gas to the wearer. The controller may control the rate of heat transfer to the reservoir of water by the heater. The controller may control the supply of electric power to the heater.
Typically the humidification device is provided within, or arranged in use to be part of, a patient circuit. The conditioning unit is a separate device from a conventional flow regulating unit, such as a ventilator. It will be appreciated to the skilled person that such flow regulating devices represent complicated and expensive assets and accordingly there is a significant benefit in providing additional functionality within the patient circuit in a manner that does not involve any adjustment to such units.
The temperature sensor may be arranged for location at, on or adjacent an internal body surface of the wearer. The internal surface may be a wall of the wearer's respiratory or digestive system such as within the trachea or oesophagus. The surface may be in the vicinity of the laryngeal framework. The wearer interface may be correspondingly shaped for location against such an internal body surface.

Such an embodiment is particularly beneficial in preventing over humidification of a gas flow for a patient having a lowered core body temperature, such as, for example, when undergoing surgery.

The wearer interface may be arranged for insertion into a wearer's airway. The wearer interface may comprise an artificial airway device. The wearer interface may comprise a tracheal tube or conduit, such as an endotracheal tube, or supraglottic airway. The wearer interface may comprise a sealing member for sealing against an internal surface of the wearer. The sealing member may be deformable to adapt to the internal surface of the wearer. The body temperature sensor may be located in a portion of the endotracheal tube or sealing member.

In embodiments in which either a body or gas temperature sensor is provided, said sensor may be encapsulated in the material of the wearer interface, typically close to, or at, its surface. The wearer interface may comprise a moulded material, within which the sensor may be encapsulated. This may be achieved by suspending the temperature sensor within a mould cavity when moulding the relevant portion of the wearer interface.

The wearer interface may be provided with one or more wires depending from the, or each, sensor. The wire may be encapsulated in the material of the wearer interface. The wearer interface may comprise a connector, for example at a portion thereof distal from the wearer and/or sensor. In one embodiment, the connector comprises an electrical connector and the wire may pass between the, or each, sensor and the connector.

The transmitter may be a wired or wireless data transmitter.

The controller may be provided within a housing of the humidification device.

Any of the sensors defined above may comprise a first sensor and the system may comprise one or more further sensors arranged to sense a characteristic of the respiratory gas flow to the wearer or else the body temperature of the wearer. The gas flow characteristic may comprise any, or any combination, of temperature, humidity, pressure and/or flow rate of the gas flow. The further sensor may also be provided within the wearer interface. Additionally or alternatively, the further sensor may be located at a location in the gas delivery system which is downstream of the flow conditioning device but upstream of the first sensor. A sensor may be located in a gas passageway at or near an inlet to the wearer interface. A sensor may be located at or near an outlet of the flow conditioning device.

In one embodiment, the controller controls the humidification device to modify the conditioning of the gas flow actively and/or automatically in response to the received data. In the event of a change to the body temperature of the wearer or the sensed flow characteristics, the control may implement a corresponding change in operation of the conditioning device. The controller may comprise a dynamic controller.

The controller may control the humidification device to heat the gas flow to the wearer to a temperature which substantially matches the body temperature measured by the sensor. The controller may set a desired value or set point for a flow conditioning parameter based upon the received body temperature data. The controller may have a memory and may have stored therein one or more preset control settings. For example, one such setting may correspond to a heating condition for a normal body temperature or a normal body temperature range. One or more settings may comprise heating conditions for elevated or depressed body temperature conditions.

Additionally or alternatively the controller may be arranged to receive sensor readings from one or more further sensors. The controller may dynamically adjust the operation of the flow conditioning device based on sensor data from the one or more further sensors in order to achieve a desired flow parameter value, which may correspond to the set point determined based upon the received body temperature data. In the example of a temperature sensor downstream of the flow conditioning device, the controller may adjust the temperature of the heater until the gas flow achieves the desired temperature at the location of the second sensor.
The controller may receive readings from any one or more of the sensors and compare the readings to previously received readings or a current flow conditioning setting. In the event that the controller determines a difference between the current readings and previous readings or settings, the controller may determine a new desired set point for the gas flow and may iteratively adjust the control of the flow conditioning device in order to achieve that new set point.
The controller may comprise machine-readable instructions, which may comprise one or more module of code and/or algorithm, for the control of any of the above described controller functions.
Further disclosed is a wearer interface for a respiratory gas delivery system, the wearer interface comprising a temperature sensor arranged to detect a body temperature of the wearer and to transmit the body temperature reading, or data corresponding thereto, to a controller of a remote flow conditioning device within the gas delivery system.

According to the invention, there is provided a respiratory gas humidifier for use in a respiratory gas delivery system, the humidifier comprising an inlet for receiving respiratory gases from a ventilator, a heater for transferring heat energy to a water reservoir, and a controller arranged to receive data signals from a core body temperature sensor located remotely of the humidifier, the controller being arranged to control operation of the heater based upon said received data signals in order to achieve a desired temperature and/or humidification of the respiratory gas supplied to a patient via the gas delivery system.

The controller may control operation of the heater such that the temperature of the respiratory gas supplied to the patient substantially matches the patient body temperature.

Further disclosed is a respiratory gas delivery system comprising the humidifier and a patient body temperature sensor.
Further disclosed is a wearer interface device for a respiratory gas delivery system, the wearer interface comprising a connector for connecting the wearer interface to a corresponding duct in the gas delivery system to allow passage of gas to the wearer via the interface, the wearer interface comprising a moulded material and having a sensor embedded in said moulded material arranged to detect a characteristic of the gas flow as it passes through the interface device, the sensor being arranged to transmit sensor reading data to a controller of a flow conditioning device within the gas delivery system.
The wearer interface may comprise a further sensor embedded in the moulded material of the interface.
Any of the optional features defined above in relation to any one aspect of the invention may be applied to any other aspect of the invention wherever practicable.
It will be appreciated to the skilled person that gas flows within a respiratory system may carry water vapour therein and/or liquid phase material, such as water, for example in the form of particles or droplets. The term "gas" should be interpreted accordingly and need not be limited to a purely gas phase fluid flow.
Practicable embodiments of the invention are described in further detail below with reference to the accompanying drawings of which:
Figure 1 shows a schematic of a system according to one embodiment of the invention;
Figure 2 shows a three-dimensional view of a patient interface according to an embodiment of the invention;
Figure 3 shows a longitudinal section through the patient interface of Figure 2;
Figure 4 shows a longitudinal section through a further patient interface according to the invention;
Figure 5 shows a longitudinal section through another patient interface according to the invention;
Figure 6 shows a humidifier for use in accordance with the present invention; and,
Figure 7 shows a flow diagram for the operation of a system according to an embodiment of the invention.

Particular instances in which the inventor has determined that conventional humidification systems may not satisfactorily adapt to patient requirements concern high-risk surgical procedures, such as cardiac or cranial surgery. The body temperature of the patient is often lowered deliberately during such procedures in order to improve patient outcome. However it has been determined that there is a risk of over humidification using conventional humidification systems when the patient's body temperature remains below normal. Such over-humidification could, if unchecked, result in a build up of liquid which could: occlude the respiratory air flow; drain back into the patient's lungs thereby putting the patient at risk of drowning; or flow into the ventilator/anaesthetic equipment thus causing damage.

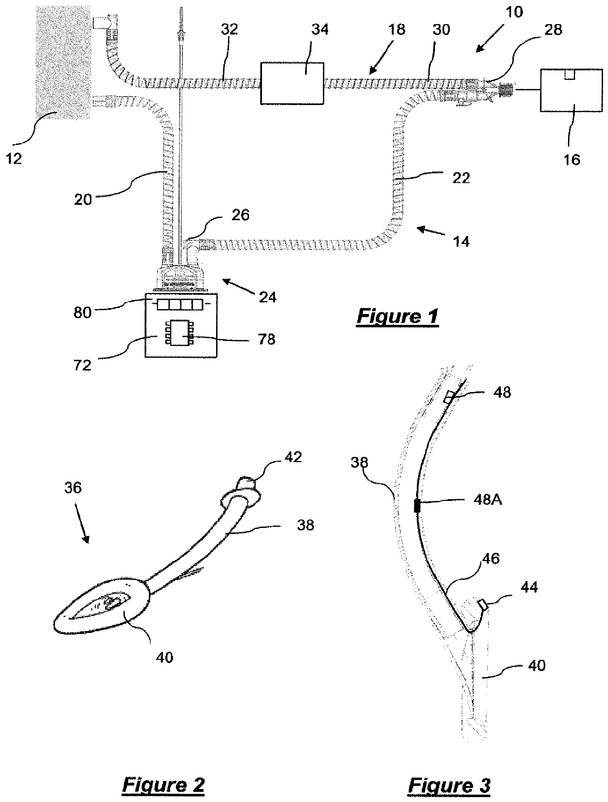

Accordingly the present invention may be considered to derive from a general premise that it is possible to actively control the operation of the humidifier in response to a sensor configuration that can better monitor the patient condition. It is also proposed that this can be achieved using a sensor located in, or connected to, the patient interface itself so as to accurately capture information on internal respiratory gas flow and core body temperature.
Whilst the system and associated method and apparatus has been developed as a result of the above-identified problems, it has since been appreciated that the features of the present invention may be more widely applied to other artificial respiratory scenarios in which there is a need to closely control the level of humidification.
Turning now to Figure 1, there is shown a respiratory system 10, which may otherwise be referred to as a breathing circuit. The system 10 comprises a conventional ventilator 12, an inspiratory limb 14 for delivering respiratory gases to a patient interface 16 for inhalation, and an expiratory limb 18 for transporting exhaled respiratory gases back to the ventilator 12. The inspiratory limb 14 comprises two breathing tubes 20, 22, the first of which is connected at one end to the ventilator 12 and the second of which leads to the patient interface 16. A humidifier 24 is connected to the inspiratory limb between the two breathing tubes 20, 22 in the flow path from the ventilator to the patient interface 16 for humidifying the respiratory gases before inhalation by the patient.

In this embodiment, breathing tube 22 disposed between the humidifier 24 and the patient interface 16 is heated, in order to maintain the temperature and humidity of the respiratory gases at a desired level for inhalation. Accordingly the tube 22 has one or more heating elements in the form of wires wound, typically helically, about the tube body. The tube has an end connector 26 comprising an electrical connector for the heating wires so as to allow connection to a power supply in use by a suitable power supply cable. The heating wire may be embedded in, co-formed with, or else attached to the tube using any conventional techniques.
Also shown in Figure 1 is a further connector 28 located between the tube 22 and the patient interface 16. The further connector takes the form of a Y-piece connector such that it provides a common connection between the patient interface and both the inspiratory 14 and expiratory 18 limbs. The Y-piece connector connects at a first port to tube 22, at a second port to the patient interface and at a third port to tube 30 of the expiratory limb. Such Y-piece connectors are generally known in the art. However in this embodiment, the Y-piece connector comprises an electrical connector formed at its connection port with the tube 22. Also the Y-piece connector forms an electrical connection with the patient interface 16, such that power and or signals can be supplied via the Y-piece connector to the patient interface as will be described below.

The expiratory limb comprises two breathing tubes 30, 32 and dehumidifying apparatus 34 connected between those two breathing tubes for removing water vapour from the exhaled respiratory gases before those respiratory gases are returned to the ventilator 12. Removal of water vapour from the exhaled respiratory gases in the expiratory limb of a breathing circuit reduces the risk of damage being caused to the ventilator by the water vapour, and also reduces the amount of condensation that occurs within the breathing tubes of the expiratory limb, which may restrict or occlude the flow passageways of the breathing tubes. In this regard a conventional dehumidifier may be used as known to the skilled person in this field.

In use, the ventilator provides a pressurized source of respiratory gas for delivery to the patient interface via the inspiratory limb, including the humidifier 24, such that the gas stream picks up moisture which is carried to the patient airway via the patient interface 16. When the patient exhales, expired gas is carried along the expiratory limb via the dehumidifier 34 back to the ventilator 12.

Exemplary embodiments of patient interfaces which may be used in accordance with the invention are described with reference to Figures 2-5 below. It is noted that the invention is well suited to invasive interface devices although it is not necessarily limited to only such devices.

Figures 2 and 3 show a first embodiment of a supraglottic airway according to the present invention, which is generally designated 36. The supraglottic airway 36 comprises a fluid conduit defined by a tubular passageway 38 and a sealing member 40 disposed at one end of the tubular passageway 38. At the other end of the tubular passageway 38, a connector 42 is provided for connecting the supraglottic airway to breathing circuit 10 via connector 28.

The sealing member 40 includes an opening that is co-extensive with the internal bore of the tubular passageway 38. The sealing member 40 is formed so that it is a snug fit over the laryngeal inlet of a patient in use, in the manner of a deformable mask member. In other embodiments, an inflatable sealing/mask member may be provided. The tubular passageway 38 and its internal bore have a generally constant cross-sections along their length, but these cross-sections increase adjacent to the sealing member, such that the opening in the sealing member 40 is enlarged relative to the internal bore of the majority of the tubular passageway.

In use, the sealing member 40 is positioned over the laryngeal framework of a patient such that sealing contact is made between the outer surface of the sealing member 40 and the entrance to the trachea.

As can be seen in Figure 3, there is provided a temperature sensor 44 as part of the component 36. In this embodiment, the temperature sensor 44 takes the form of a thermistor, typically a conventional bead-type thermistor. The sensor 44 is provided within the body material of the patient interface adjacent an outer surface of the interface which is intended to contact an internal surface of the patient in use. In this embodiment, the sensor 44 is mounted within the material of the sealing member, although in other embodiments, the sensor may be mounted at alternative locations within the wall structure of the device. In particular the sensor may be mounted in a tip region of the device. In use, the sensor 44 is located immediately beneath the surface of the device and held adjacent, in thermal contact with, tissues in the vicinity of the junction between the oesophagus and trachea.

Two wires 46 depend from the sensor 44 and pass along the wall to the connector 42 end of the device 36. An electrical connector (not shown), typically in the form of a two-pin electrical connector is provided to allow electrical connection with a corresponding connector formation depending from the Y-piece connector 28. The wires 46 are embedded or encapsulated within the wall material of the device.

Also shown is Figure 3 is a further sensor 48 which may be provided instead of or else in addition to the sensor 44. The further sensor 48 may be arranged to measure one or more characteristics of the flow along the internal bore of the device 36. Thus the sensor 48 is mounted at an internal surface defining the flow passageway along the device, rather than against an outer surface of the device as is the case for sensor 44. The sensor 48 may be a conventional temperature sensor for measure fluid temperature flowing there-over or else a flow rate sensor, the purpose of which will be described in further detail below. The sensor 48 is provided towards the end (i.e. the connector end) of the device which opposes the sealing member 40. That end of the device is typically spaced from the internal surfaces of the patient's body in use and thus allows recording of fluid flow characteristics substantially in isolation of the patient's body temperature. However the sensor, or a further sensor, could equally be provided part-way or mid-way along the tube 38 as shown at 48A.

During manufacture, the, or each sensor, including any associated wires, is held within a mould in which the device 36 is formed. The mould may have two cavity portions for defining each of the passageway 38 and seal 40 portions of the device 36. The sensor is typically held adjacent or fractionally spaced from the relevant mould cavity wall. The tubular passageway 38 is then formed in the first cavity portion by injection moulding. The sealing member 40 is then formed by a second, subsequent injection moulding step onto the tubular passageway 38, as part of a so-called two-shot injection moulding process. Depending on the location of the sensor(s) in the component 36, the sensor may be held in the first of second mould cavity portion as necessary. However it is generally preferred that the sensor is mounted in a thicker material portion of the component, such as within the sealing member or else in the vicinity of the connector end so as to easily accommodate the sensor within the wall of the device.

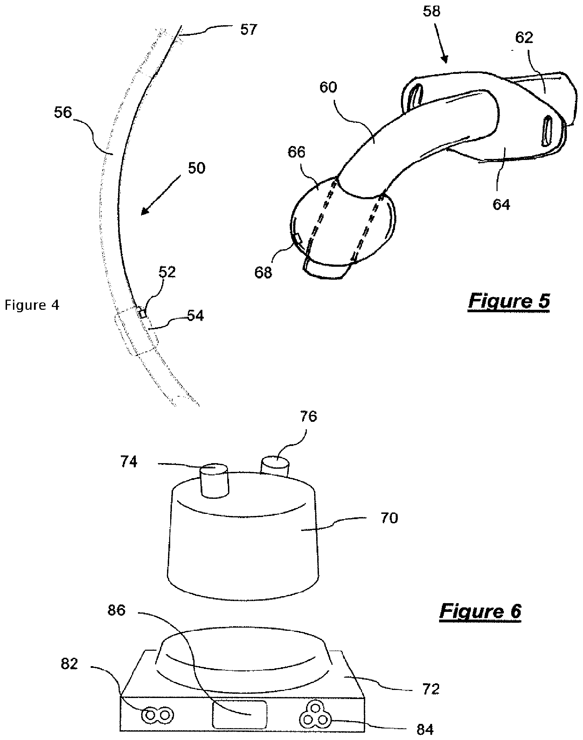

Figure 4 shows a second embodiment of an artificial airway according to the present invention, which is generally designated 50. This embodiment of patient interface takes the form of an endotracheal tube. The artificial airway 50 includes at least one sensor 52 mounted/embedded therein in a manner which is similar to the device described in relation to Figure 3 and may be formed for example by an injection moulding process. However, in line with the intended use of an endotracheal tube, a different form of the sealing member 54 and its attachment to the tubular passageway 56 is provided. In this embodiment, the sealing member 54 is generally annular in section and takes the form of a sealing cuff around the tube 56. As will be appreciated by the skilled person, the sealing member 54 is intended to contact, and thereby seal with, the internal wall of a patient's airway in use, albeit at a different location to that of the laryngeal mask of Figures 2 and 3. Accordingly the sensor location(s) and connection with the connector formation 57 may be as described above.

Turning now to Figure 5, there is shown another type of patient interface in the form of a tracheostomy tube arrangement 58 in accordance with the present invention. The tracheostomy tube 58 comprises a fluid conduit 60 which is arcuate in form, having a generally tubular internal passageway. The tubular passageway includes a connector 62 at one end that is adapted to connect the tracheostomy tube to the breathing circuit. The tracheostomy tube also includes a flange 64, which is disposed adjacent to the connector 62. The flange includes an opening at each end for engagement with a strap (not shown in the Figures), which secures the tracheostomy tube 10 to the patient.

At the other end of the tubular passageway 60, is a sealing member 66 which surrounds a portion of the tubular passageway. The sealing member has a circular cross-section, and an external surface that is generally convex in form along its longitudinal axis. The sealing member 66 has a temperature sensor 68 mounted towards its outer surface in a manner as described above. The skilled person will appreciate that, other than its shape and appearance, the patient interface 58 may be formed in a manner as described above using similar materials and manufacturing techniques, such that the patient interface 58 may also comprise any of the different sensor arrangements described in relation to Figures 3 and 4.

Any of the temperature sensors 44, 52 or 68 described above can be located in use against an internal bodily surface of a patient such that those sensors can operate to measure the patient's core body temperature. Additionally or alternatively, the one or more sensors 48 mounted at an interior surface of the patient interface can measure gas temperature or flow rate of the fluid flow to/from the patient's lungs in-situ. The proximity of the point of measurement of such fluid flow characteristics to the patient's lungs provides can provide greater accuracy and certainty than has been hitherto possible. Also the mode of manufacture described above provides for a cost-effective and unobtrusive sensor configuration.

Turning now to Figures 1 and 6, there is shown a humidification device 24 for use in accordance with the improved respiratory system. The humidifier 24 comprises an assembly of a disposable reservoir, or water chamber, 70 and a base unit 72 on which the reservoir 70 is positioned in use. The reservoir comprises an enclosure having a generally hollow interior, which is part-filled with water in use, and an inlet 74 and outlet 76 port above the level of the water to allow the flow of air into and from the reservoir 70 such that the air passes over the surface of the water therein. Various other additional features of the reservoir will be known to the skilled person in this field and will not be described here for conciseness.

The base unit 72 has a controller 78 and a heater element 80 as shown in Figure 1. The controller comprises a programmable chip having machine readable instructions for the processing of received data and the outputting control signals in a manner to be described below. The controller is principally used to control the operation of the heater and thereby control the rate of heating and temperature of the water reservoir 70. The reservoir 70 typically has a metallic base wall component for contact with an upper heater surface of the base unit 72 to allow effective heat transfer there-between.

The base unit 72 comprises two separate electrical connectors 82 and 84 for respective connection to a power supply and the patient interface 16. The connectors are shown as a two-pin and three-pin connector respectively but could comprise any, or any combination, of two and three-pin connectors to suit the desired system. The connection with the patient interface in this embodiment is achieved by a wired connection to the connector 26 in tube 22 and thereby to the patient interface connector via the Y-piece 28. Whilst such an arrangement is in many ways preferred, in alternative embodiments a direct wired connection could potentially be provided from the base to the interface connector which bypasses the intermediate members.

A wired connection allows the supply of power from the base unit to the patient interface. The wired connection also allows transmission of sensor readings in the form of data signals to the controller 78.

In alternative embodiments, it will be appreciated that the sensor data could be sent to the controller via a wireless data connection, for example using a suitable standard for data transfer over a radio signal. Either Bluetooth (RTM) or Wi-Fi (RTM) may be suitable for this purpose and the additional electronic components could be embedded in the moulded patient interface component in a manner described above. Accordingly a transmitter and battery could be provided at a suitable location in the patient interface or else in another intermediate connector within the system, such as within the Y-piece connector 28 for connection to the patient interface sensor(s). In either the wired or wireless embodiments, the controller circuitry will typically comprise a receiver for the relevant data signals.

As a further example of the invention, a temperature sensor could be provided to measure the patient's body temperature at a location remote from the patient interface. For example an in-ear temperature sensor or skin pad/patch sensor could be attached to the patient interface connector by a flexible wired connection. In ear and/or skin temperature sensors are considered generally preferable in this regard since they can be located close to the patient interface in use and thus require only short electrical leads to the patient interface. However if a wireless temperature sensor is used, then a wide variety of sensor locations on the patient's body could be accommodated.

Also shown in Figure 6 is a display 86 on the base unit 72, which may be used to display information on the current operational state to an operator. The display may have multiple functions and may display any or any combination of, for example: the temperature of the water reservoir; the temperature of the respiratory flow at the patient interface; the core body temperature of the patient; a temperature differential between the water reservoir and the patient; a flow rate at the patient interface. The base unit may be configured to provide a visual and/or audible alarm if any of those parameters are determined to fall outside of an acceptable operating range.

It will be appreciated that the system may comprise additional flow and/or temperature sensors, such as for example at, or shortly downstream of, the outlet 76 of the humidifier. Also in some embodiments, it will be appreciated that a temperature and/or flow sensor could be provided immediately upstream of the patient interface, for example within the tube 22 connector or else in the Y-piece connector 28, rather than being embedded in the patient interface itself.

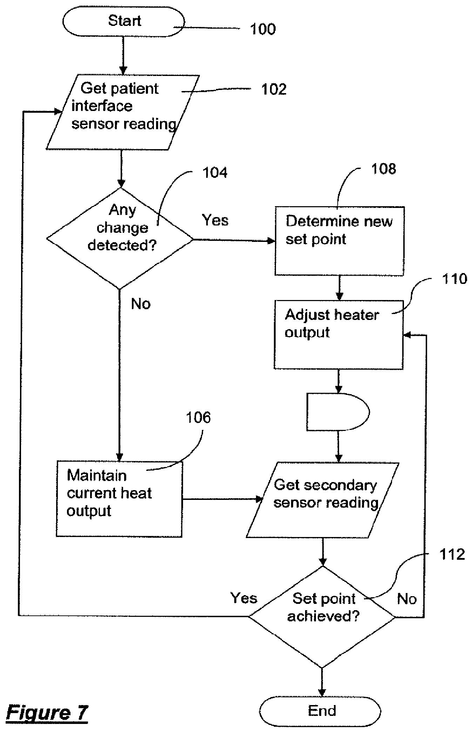

Turning now to Figure 7 there is shown a flow diagram of the operational steps undertaken in order to facilitate active, responsive control of the humidifier 24. At 100 the controller enters a start-up mode, where the controller will typically check the relevant sensor connections and initiate heating of the water reservoir in a predetermined or fixed manner.

During operation of the system 10, the one or more sensors described above take readings of the corresponding operational variables at a suitable frequency and resulting data signals are transmitted to the controller 78. Signals are typically transmitted intermittently at predetermined time intervals. Alternatively, the controller could actively initiate signal transmission in a two-way communication embodiment.

At 102 the controller receives the sensor data signal. In this embodiment, with reference to the patient interface of Figure 3, the controller receives at least the reading from the core body temperature sensor 44. The controller compares the received signal with previously stored data at 104 to determine whether there has been any change in the sensed parameter. In this regard, the controller typically has a predetermined initial value which corresponds to a normal body temperature of approximately 37°C, which is updated as sensor readings are received in use.

The controller also has an initial desired operating condition which it aims to achieve. The desired operating condition in this embodiment relates to a desired gas delivery temperature at the patient interface, which is determined by sensor 48. The reading from this sensor is used by the controller to determine whether the desired operating condition or set point has been achieved.

In the event that no change is detected at 104, the current heater operation state or mode is maintained at 106. Conversely if a change in the sensor reading is detected, which in this embodiment corresponds to a change in patient core body temperature, the controller determines a new desired set point at 108. This new set point corresponds to a desired inspiratory gas delivery temperature at the patient interface. The controller then adjusts the operation of the heater at 110, typically by modifying the supply of electrical power to the heating element 80, in order to achieve this desired set point.

The controller may wait a short time to allow the change in heater setting to take effect before processing a data signal from the second sensor 48 in order to compare that reading with the current set point so as to determine whether the gas supply has achieved the desired set point at the patient interface. The desired set point may equal the body temperature sensed by the sensor 44 such that the gas supply inspired by the patient matches their body temperature. If the desired gas delivery condition is not achieved, the controller returns to step 110 and applies a further heater control adjustment before repeating the steps to determine whether the set point has been achieved. This loop is repeated as often as required to achieve the desired level of operation.

If the desired gas delivery condition is achieved at 112, the controller may then return to step 102 so as to operate a loop in which the above steps are repeated in suitable time increments.

Accordingly the delivery of respiratory gas is actively controlled based on body temperature. The controller may be programmed to respond only to differences in sensor readings above a predetermined magnitude. Thus the controller need not constantly update the operation of the humidifier in response to minor fluctuations in operating conditions as may be typical in normal use. The controller may comprise a timer such that the controller can determine a rate of change of sensor readings and control operation of the humidifier accordingly.

In a simplified embodiment, the further sensor 48 in the patient interface may not be provided and instead the controller can control the heater to heat the water reservoir to achieve a desired outlet temperature at port 76. The controller in this embodiment can apply a predetermined temperature loss factor to approximate the delivery temperature of the inspiratory gas at the patient interface without measuring it directly. Such an embodiment may provide a simplified system, albeit at the expense of possible reduction in responsiveness or accuracy.

In a further alternative configuration, the core body temperature sensor may not be provided and instead, only the gas flow condition sensor may be provided in the patient interface. In such an embodiment, the controller could receive flow temperature readings at a frequency that is greater than or equal to the respiratory rate of the patient in order to determine a temperature differential between inspiration and expiration. Thus the controller could control operation of the heater in response to sensor readings in order to achieve a condition where the inspiratory temperature substantially matches the expiratory temperature. Such an embodiment may require a greater temperature reading resolution and frequency than the embodiments which rely on two or more sensors in combination.

In any of the above embodiments, it may be possible to substitute different types of sensors in the gas path such that, for example, a flow rate sensor may be used to provide a further indicator of correct functioning of the system. Additionally or alternatively, a humidity sensor could be located in the gas path to provide direct humidity measurements, which can be calibrated to provide suitable set points for given body temperatures.

In view of the above described embodiments, it will be appreciated that a considerable advantage in control and responsiveness of the system can be achieved by locating one or more suitable senor(s) at the patient interface.

As a further development, in the event that a heated tube 22 is used, it is proposed that the controller 78 may additionally or alternatively control the supply of power to the heating wire for the tube in the manner described above so as to ensure that a desired delivery temperature of the respiratory gas is achieved on the inspiratory limb.

## Claims

1. A respiratory gas humidifier (24) for use in a respiratory gas delivery system (10), the humidifier (24) comprising an inlet (74) for receiving respiratory gases from a separate ventilator (12), a heater (80) for transferring heat energy to a water reservoir (70), and a controller (78) configured to receive data signals from a core body temperature sensor (44) located remotely of the humidifier (24), the controller (78) being configured to control operation of the heater (80) based upon said received data signals in order to achieve a desired temperature and/or humidification of the respiratory gas supplied to a patient via the gas delivery system (10).

2. A humidifier (24) according to claim 1, wherein the controller (78) is configured to receive one or more further sensor data signals and to compare said further data signal with said desired temperature and/or humidification in order to determine whether said desired temperature and/or humidification has been achieved.

3. A respiratory gas delivery system (10) comprising:
a wearer interface (16) through which respiratory gas is supplied to a wearer's airway, the wearer interface comprising a sensor (44) for measuring the core body temperature of the wearer;
a ventilator; and
a respiratory gas humidifier (24) according to claim 1 or claim 2, the respiratory gas humidifier (24) being a separate device to the ventilator.

4. A system according to claim 3, wherein the interface (16) comprises a moulded material and the core body temperature sensor (44) is embedded within said moulded material in a portion of said wearer interface (16) that is inserted into a wearer's airway in use.

5. A system according to claim 4, wherein the wearer interface (58) comprises a supraglottic airway (36) or an endotracheal tube (56) having a compliant sealing member (40,54) for sealing with the trachea of the wearer in use, wherein the body temperature sensor (44) is located in the sealing member.

6. A system according to claim 3, wherein the heater (80) and controller (78) are housed in a base unit (72) of the humidifier (24), the base unit further comprising an electrical connector (84) for electrical connection with the patient interface (16).

7. A system according to any of claims 3 to 6 comprising an intermediate conduit (22) for the delivery of respiratory gas from the gas conditioning device (72) to the wearer interface (16), the conduit comprising an electrical connector at each end thereof and a conducting member connected there-between so as to allow an electrical connection between the conditioning device (72) and the wearer interface (16).

8. A system according to any of claims 3 to 7, wherein the interface (58) comprises a first end (60) for insertion into a wearer's trachea in use and an opposing end (62), the interface comprising an electrical connector in the vicinity of said opposing end (42) and one or more wires (46) extending between the sensor (44) and the connector.

9. A system according to any of claims 3 to 8 wherein the sensor (44) has a corresponding data transmitter arranged to transmit sensor readings data to the controller (78).

10. A system according to any of claims 3 to 9, wherein the core body temperature sensor (44) comprises a first sensor and the system also comprises one or more further sensors arranged to sense a characteristic of the respiratory gas flow to or from the wearer.

11. A system according to claim 10, wherein the further sensor comprises a gas temperature sensor.

12. A system according to any of claims 3 to 11 wherein the controller (78) is arranged to compare the received sensor measurement to a preceding sensor measurement or a predetermined value and to control the operation of the heater (80) automatically upon determination of a difference between the received sensor measurement and said preceding measurement or predetermined value.

13. A system according to any of claims 3 to 12, wherein the controller (78) sets a desired operational variable value based on said received sensor measurement.

14. A system according to claim 13, when dependent on any one of claims 9 to 10, wherein the controller (78) determines whether the set point has been reached based on readings from said one or more further sensors.

## Patentansprüche

1. Atemgasbefeuchter (24) zur Verwendung in einem Atemgasverabreichungssystem (10), wobei der Befeuchter (24) einen Einlass (74) zum Aufnehmen von Atemgasen aus einer separaten Beatmungseinheit (12), ein Heizelement (80) zum Übertragen von Wärmeenergie auf einen Wasserbehälter (70) und eine Steuerung (78) umfasst, die dazu konfiguriert ist, Datensignale von einem Körperkerntemperatursensor (44) zu empfangen, der entfernt vom Befeuchter (24) angeordnet ist, wobei die Steuerung (78) dazu konfiguriert ist, den Betrieb des Heizelements (80) auf Grundlage von den empfangenen Datensignalen zu steuern, um eine gewünschte Temperatur und/oder Befeuchtung des Atemgases, das einem Patienten über das Gasverabreichungssystem (10) zugeführt wird, zu erreichen.

2. Befeuchter (24) nach Anspruch 1, wobei die Steuerung (78) dazu konfiguriert ist, ein oder mehrere weitere Sensordatensignale zu empfangen und die weiteren Datensignale mit der gewünschten Temperatur und/oder Befeuchtung zu vergleichen, um festzustellen, ob die gewünschte Temperatur und/oder Befeuchtung erreicht wurde.

3. Atemgas-Verabreichungssystem (10), umfassend:
eine Trägerschnittstelle (16), durch die den Atemwegen des Trägers Atemgas zugeführt wird, wobei die Trägerschnittstelle einen Sensor (44) zum Messen der Körperkerntemperatur des Trägers umfasst; eine Beatmungseinheit; und
einen Atemgasbefeuchter (24) nach Anspruch 1 oder Anspruch 2, wobei es sich beim Atemgasbefeuchter (24) um eine separate Vorrichtung der Beatmungseinheit handelt.

4. System nach Anspruch 3, wobei die Schnittstelle (16) ein geformtes Material umfasst und der Körperkerntemperatursensor (44) in das geformte Material in einem Abschnitt der Trägerschnittstelle (16) eingebettet ist, das bei Verwendung in die Atemwege des Trägers eingeführt wird.

5. System nach Anspruch 4, wobei die Trägerschnittstelle (58) eine supraglottische Atemwegshilfe (36) oder einen Endotrachealtubus (56) umfasst, die ein nachgiebiges Dichtungselement (40, 54) zum Abdichten der Luftröhre des Trägers bei Verwendung aufweisen, wobei der Körpertemperatursensor (44) sich im Dichtungselement befindet.

6. System nach Anspruch 3, wobei sich das Heizelement (80) und die Steuerung (78) in einer Basiseinheit (72) des Befeuchters (24) befinden, wobei die Basiseinheit ferner einen elektrischen Verbinder (84) zur elektrischen Verbindung mit der Patientenschnittstelle (16) umfasst.

7. System nach einem der Ansprüche 3 bis 6, umfassend eine Zwischenleitung (22) für die Verabreichung von Atemgas von der Gasbehandlungsvorrichtung (72) zur Trägerschnittstelle (16), wobei die Leitung an jedem ihrer Enden einen elektrischen Verbinder und ein leitendes Element dazwischen umfasst, damit eine elektrische Verbindung zwischen der Behandlungsvorrichtung (72) und der Trägerschnittstelle (16) ermöglicht wird.

8. System nach einem der Ansprüche 3 bis 7, wobei die Schnittstelle (58) ein erstes Ende (60) zur Einführung in die Luftröhre des Trägers in Verwendung und ein entgegengesetztes Ende (62) umfasst, wobei die Schnittstelle einen elektrischen Verbinder in der Nähe des entgegengesetzten Endes (42) und einen oder mehrere Kabel (46) umfasst, die sich zwischen dem Sensor (44) und dem Verbinder erstrecken.

9. System nach einem der Ansprüche 3 bis 8, wobei der Sensor (44) über einen entsprechenden Datensender verfügt, der eingerichtet ist, um Sensormesswerte an die Steuerung (78) zu übertragen.

10. System nach einem der Ansprüche 3 bis 9, wobei der Körperkerntemperatursensor (44) einen ersten Sensor umfasst und das System ebenfalls einen oder mehrere weitere Sensoren umfasst, die angeordnet sind, um eine Eigenschaft des Atemgasflusses zu oder von dem Träger zu erfassen.

11. System nach Anspruch 10, wobei der weitere Sensor einen Gastemperatursensor umfasst.

12. System nach einem der Ansprüche 3 bis 11, wobei die Steuerung (78) angeordnet ist, um die empfangene Sensormessung mit einer vorhergehenden Sensormessung oder einem vorbestimmten Wert zu vergleichen und um den Betrieb des Heizelements (80) bei Feststellung einer Differenz zwischen der empfangenen Sensormessung und der vorhergehenden Messung oder dem vorbestimmten Wert automatisch zu steuern.

13. System nach Anspruch 3 bis 12, wobei die Steuerung (78) einen gewünschten variablen Betriebswert auf Grundlage der empfangenen Sensormessung festlegt.

14. System nach Anspruch 13 in Abhängigkeit von einem der Ansprüche 9 bis 10, wobei die Steuerung (78) auf Grundlage von Messwerten von dem einen oder mehreren weiteren Sensoren bestimmt, ob der Sollwert erreicht wurde.

## Revendications

1. Humidificateur de gaz respiratoire (24) destiné à être utilisé dans un système d'administration de gaz respiratoire (10), ledit humidificateur (24) comprenant une entrée (74) permettant de recevoir des gaz respiratoires depuis un ventilateur distinct (12), un dispositif de chauffage (80) permettant de transférer de l'énergie thermique à un réservoir d'eau (70), et un dispositif de réglage (78) conçu pour recevoir des signaux de données d'un capteur de température interne de corps (44) situé à distance de l'humidificateur (24), ledit dispositif de réglage (78) étant conçu pour régler le fonctionnement du dispositif de chauffage (80) sur la base desdits signaux de données reçus afin d'atteindre une température souhaitée et/ou une humidification souhaitée du gaz respiratoire fourni à un patient par l'intermédiaire du système d'administration de gaz (10).

2. Humidificateur (24) selon la revendication 1, ledit dispositif de réglage (78) étant conçu pour recevoir un ou plusieurs signaux de données de capteur supplémentaires et pour comparer ledit signal de données supplémentaire avec ladite température souhaitée et/ou ladite humidification souhaitée afin de déterminer si ladite la température souhaitée et/ou ladite humidification souhaitée ont été atteintes.

3. Système d'administration de gaz respiratoire (10) comprenant :
une interface d'utilisateur (16) à travers laquelle le gaz respiratoire est administré dans les voies aériennes de l'utilisateur, ladite interface d'utilisateur comprenant un capteur (44) permettant de mesurer la température interne de corps de l'utilisateur ; un ventilateur ; et
un humidificateur de gaz respiratoire (24) selon la revendication 1 ou 2, ledit humidificateur de gaz respiratoire (24) étant un dispositif distinct du ventilateur.

4. Système selon la revendication 3, ladite interface (16) comprenant un matériau moulé et ledit capteur de température interne de corps (44) étant incorporé dans ledit matériau moulé dans une partie de ladite interface d'utilisateur (16) qui est insérée dans les voies aériennes de l'utilisateur lors de l'utilisation.

5. Système selon la revendication 4, ladite interface d'utilisateur (58) comprenant une voie aérienne supraglottique (36) ou une sonde endotrachéale (56) possédant un élément d'étanchéité conforme (40, 54) permettant d'assurer l'étanchéité avec la trachée de l'utilisateur lors de l'utilisation, ledit capteur de température de corps (44) étant situé dans l'élément d'étanchéité.

6. Système selon la revendication 3, ledit dispositif de chauffage (80) et ledit dispositif de réglage (78) étant logés dans une unité de base (72) de l'humidificateur (24), ladite unité de base comprenant en outre un raccord électrique (84) permettant une liaison électrique avec l'interface du patient (16).

7. Système selon l'une quelconque des revendications 3 à 6, comprenant un conduit intermédiaire (22) permettant l'administration du gaz respiratoire allant du dispositif de conditionnement de gaz (72) jusqu'à l'interface d'utilisateur (16), ledit conduit comprenant un raccord électrique à chaque extrémité de celui-ci et un élément conducteur les reliant afin de permettre une liaison électrique entre le dispositif de conditionnement (72) et l'interface d'utilisateur (16).

8. Système selon l'une quelconque des revendications 3 à 7, ladite interface (58) comprenant une première extrémité (60) permettant une insertion dans la trachée de l'utilisateur lors de l'utilisation et une extrémité opposée (62), ladite interface comprenant un raccord électrique dans le voisinage de ladite extrémité opposée (42) et un ou plusieurs fils métalliques (46) s'étendant entre le capteur (44) et le raccord.

9. Système selon l'une quelconque des revendications 3 à 8, ledit capteur (44) possédant un émetteur de données correspondant agencé pour transmettre des données de lecture de capteur au dispositif de réglage (78).

10. Système selon l'une quelconque des revendications 3 à 9, ledit capteur de température interne de corps (44) comprenant un premier capteur et ledit système comprenant aussi un ou plusieurs capteurs supplémentaires agencés pour détecter une caractéristique du flux de gaz respiratoire vers ou en provenance de l'utilisateur.

11. Système selon la revendication 10, ledit capteur supplémentaire comprenant un capteur de température de gaz.

12. Système selon l'une quelconque des revendications 3 à 11, ledit dispositif de réglage (78) étant agencé pour comparer la mesure de capteur reçue à une mesure de capteur précédente ou à une valeur préétablie et à régler le fonctionnement du dispositif de chauffage (80) automatiquement en réponse à la détermination d'une différence entre la mesure de capteur reçue et la ladite mesure précédente ou ladite valeur préétablie.

13. Système selon l'une quelconque des revendications 3 à 12, ledit dispositif de réglage (78) définissant une valeur variable de fonctionnement souhaitée sur la base de ladite mesure de capteur reçue.

14. Système selon la revendication 13, lorsque dépendante de l'une quelconque des revendications 9 à 10, ledit dispositif de réglage (78) déterminant si la valeur de consigne a été atteinte sur la base des lectures provenant dudit ou desdits capteurs supplémentaires.
